# EUROPEAN PATENT APPLICATION

(11) **EP 4 835 082 A1**
(43) Date of publication of application: **04.11.2026**
(21) Application number: 24912642.6
(22) Date of filing: 19.12.2024
(51) Int. Cl.: C12N 15/53, C07K 14/195, C12N 1/21, C12P 7/04

(54) **CROTONALDEHYDE-CROTYL ALCOHOL DEHYDROGENASE MUTANT**

(30) Priority: 27.12.2023 JP 2023222025
(71) Applicant: Utilization of Carbon Dioxide Institute Co.,Ltd., Tokyo 135-0091 (JP); ENEOS Materials Corporation, Tokyo 105-7109 (JP)
(72) Inventor: YUKAWA, Hideaki, Tokyo 135-0091 (JP)
(74) Representative: Müller-Boré & Partner Patentanwälte PartG mbB
(86) International application number: PCT/JP2024/045077
(87) International publication number: WO 2025/142753

(57) **Abstract**

A bacterium with a gene introduced, the gene encoding a crotonaldehyde-crotyl alcohol dehydrogenase mutant described in the following (1) or (2) is able to efficiently produce crotyl alcohol which is used as a raw material for producing butadiene.
(1) A DNA comprising a nucleotide sequence having, in the nucleotide sequence of SEQ ID NO: 1, a nucleotide substitution resulting in a substitution of isoleucine at position 403 of SEQ ID NO: 2 encoded by SEQ ID NO: 1 with serine, glycine, methionine, alanine, proline, glutamine, phenylalanine, leucine, tryptophan, lysine, cysteine, or glutamic acid
(2) A DNA encoding a polypeptide described in the following (2A) or (2B):
(2A) a polypeptide comprising an amino acid sequence that has 90% or more identity to an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid), or
(2B) a polypeptide comprising an amino acid sequence that has 1 to 80 amino acids deleted, substituted, inserted, or added in an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid)

## Description

### Technical Field

The present invention relates to a crotonaldehyde-crotyl alcohol dehydrogenase mutant of which ability of producing crotyl alcohol from crotonyl-CoA is enhanced, a gene encoding the mutant, a transformant produced by introducing the gene, and a method of producing crotyl alcohol using the transformant.

### Background Art

The Paris Agreement, which was adopted in 2015, provides that global emissions of greenhouse gases should be promptly reduced. Under the Agreement, Japan set the goal of reducing the emission of greenhouse gases such as carbon dioxide and methane by 46% compared with year 2013 levels, by the year 2030.

Worldwide, the majority of the production of chemicals depends on petroleum resources, exacerbating the problem of increased greenhouse gas emissions. Accordingly, departure from petroleum dependency is a desirable strategy for the production of chemicals, and research and development of biorefineries that produce green chemicals from biomass is being earnestly carried out in various countries. However, the saccharification of biomass for use as raw materials of microbial fermentation necessitates complex processes, besides being costly. Using biomass that could serve as food or feed for chemical production disturbs the stable supply of food and feed. Furthermore, the large-scale consumption of biomass for chemical manufacturing raises concerns that it actually leads to environmental destruction.

As part of research geared towards departure from petroleum dependency, gases such as carbon dioxide, methane, and carbon monoxide have attracted attention as more sustainable carbon sources, and techniques for producing valuable chemicals and biofuels using microorganisms that utilize these gases are the subject of interest. In particular, carbon fixation and efficient utilization of carbon dioxide, a significant contributor to global warming, is highly anticipated.

Butadiene is used as a raw material for synthetic rubbers such as butadiene rubber, styrene-butadiene rubber, acrylonitrile-butadiene rubber, and chloroprene rubber, as well as for synthetic resins such as acrylonitrile-butadiene-styrene (ABS) resin and methyl methacrylate-butadiene-styrene (MBS) resin. It is also utilized as a precursor for various other chemical products, including adiponitrile, 1,4-butanediol, cyclododecatriene, chloroprene, and sulfolane.

Most butadiene is currently produced using petrochemical techniques, and there is a need to reduce dependence on petroleum resources and improve sustainability. Accordingly, attention has been directed to bio-butadiene, which is butadiene produced from bio-based raw materials. Although methods for synthesizing butadiene from ethanol have been known since as early as the 1940s, they lost their competitiveness due to the establishment of distillation techniques for extracting butadiene from the C4 fraction obtained by naphtha steam cracking and consequently fell largely into disuse.

In recent years, however, the production of butadiene from bio-ethanol, which is ethanol derived from biological sources, has gained renewed attention. Most recently, a bio-butadiene production technique using a four-step continuous process has been reported (Non Patent Literature 1). The four-step continuous process is composed of the first-step reaction in which acetaldehyde is produced by dehydrogenation of ethanol; the second-step reaction in which crotonaldehyde is produced by aldol condensation of acetaldehyde; the third-step reaction in which crotyl alcohol is produced by selective hydrogenation of crotonaldehyde; and the fourth-step reaction in which butadiene is produced by dehydration of crotyl alcohol. Although an approach using bio-ethanol as the starting material ethanol in the four-step continuous process has also been employed, it does not sufficiently reduce the environmental burden because the conversion of ethanol to butadiene requires chemical reactions.

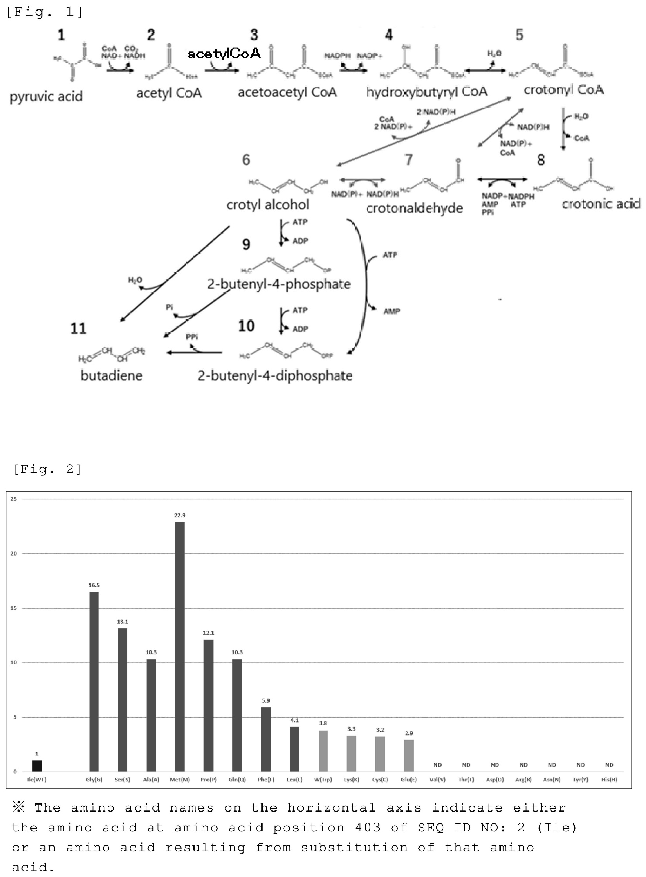

Consequently, techniques for producing butadiene using microorganisms have been explored. Many microorganisms have a metabolic pathway that produces butadiene from pyruvate via crotonyl-CoA and crotyl alcohol (crotonyl alcohol) as shown in Fig. 1; however, they generally lack the ability to produce butadiene on an industrial scale. Therefore, various methods have been proposed for producing butadiene by culturing transformants obtained by introducing, into hosts, one or more exogenous genes encoding enzymes that constitute this metabolic pathway.

For example, Patent Literature 1 discloses a method for producing butadiene that comprises a step of generating crotyl alcohol by culturing an *Escherichia coli* transformant, and a subsequent step of chemically converting the crotyl alcohol into butadiene (Claim 1). The transformant used in this method is engineered by introducing the following exogenous genes (A) and (B) encoding enzymes that catalyze the conversion from crotonyl-CoA to crotyl alcohol:
(A) One of the following (1) to (3):
   (1) An aldehyde-forming crotonyl-CoA reductase (Compound 5 to 7 in Figure 1) and an alcohol-forming crotonaldehyde reductase (Compound 7 to 6 in Figure 1)
   (2) An alcohol-forming crotonyl-CoA reductase (Compound 5 to 6 in Figure 1)
   (3) A crotonyl-CoA hydrolase (Compound 5 to 8 in Figure 1), crotonyl-CoA synthetase (Compound 5 to 8 in Figure 1), or crotonyl-CoA transferase (Compound 5 to 8 in Figure 1), together with a crotonic acid reductase (Compound 8 to 7 in Figure 1) and an alcohol-forming crotonaldehyde reductase (Compound 7 to 6 in Figure 1).
(B) Acetyl-CoA:acetyl-CoA acyltransferase (Compound 2 to 3 in Figure 1), acetoacetyl-CoA reductase (Compound 3 to 4 in Figure 1), and 3-hydroxybutyryl-CoA dehydratase (Compound 4 to 5 in Figure 1)

However, the method described in Patent Literature 1 uses glucose or starch derived from biomass as a carbon source (paragraph 0087). As noted above, the use of biomass involves complex processes to convert biomass into sugars, which increases production costs. Moreover, the industrial use of biomass actually causes environmental load.

Patent Literature 2 discloses a method for producing butadiene by bringing a transformant in contact with a carbon source in a culture medium (Claims 1 and 2), the transformant being prepared by introducing following exogenous gene (A) or (B) into a bacterium belonging to the genera *Burkholderia*, *Propionibacterium*, *Propionispira*, *Clostridium*, *Bacillus*, *Escherichia*, *Pelobacter*, or *Lactobacillus*.
(A) A crotonyl-CoA reductase (Compound 5 to 7 in Figure 1) and a linalool dehydratase, which is a type of crotonyl alcohol dehydratase (Compound 6 to 11 in Figure 1)
(B) A crotonaldehyde dehydrogenase (Compound 5 to 7 in Figure 1), a crotonyl alcohol dehydrogenase (Compound 7 to 6 in Figure 1), and a linalool dehydratase, which is a type of crotonyl alcohol dehydratase (Compound 6 to 11 in Figure 1).

However, the method described in Patent Literature 2 produces butadiene by cultivating the above microorganisms using sugars or similar compounds as carbon sources (e.g., paragraph 0088), and has disadvantages accompanied by use of biomass.

A method for producing crotyl alcohol, a precursor of butadiene, using microorganisms has also been proposed.

For example, Patent Literature 3 discloses a technique for producing crotyl alcohol from one or more C1 compounds selected from methane, methanol, methylamine, formic acid, formaldehyde, and formamide with use of a transformant (Claim 11 and paragraph 0001). The transformant is prepared by introducing at least one gene selected from a gene encoding acetoacetyl-CoA thiolase (Compound 2 to 3 in Figure 1), a gene encoding 3-hydroxybutyryl-CoA dehydrogenase (Compound 3 to 4 in Figure 1), a gene encoding 3-hydroxybutyryl-CoA dehydratase (Compound 4 to 5 in Figure 1), a gene encoding crotonyl-CoA hydrolase (Compound 5 to 8 in Figure 1), a gene encoding crotonic acid reductase Compound 8 to 7 in Figure 1), and a gene encoding alcohol-forming crotonaldehyde reductase (Compound 7 to 6 in Figure 1) as well as genes encoding enzymes that convert methanol and/or formic acid into formaldehyde and genes encoding enzymes that confer formaldehyde fixation capability, into a host, a methylotroph such as methylotrophic yeast.

However, C1 compounds such as methane are primarily synthesized with use of natural gas, coal, oil shale, and the like as raw materials. Therefore, the method described in Patent Literature 3 cannot be considered effective in reducing greenhouse gas emissions.

### Citation List

### Patent Literature

[Patent Literature 1] JP5960729B
[Patent Literature 2] JP6415326B
[Patent Literature 3] JP2014-155455A

### Non Patent Literature

[Non Patent Literature 1] Journal of the Japan Petroleum Institute, 63, (2), 70-78(2020)

### Summary of Invention

### Technical Problem

An objective of the present invention is to provide a crotonaldehyde-crotyl alcohol dehydrogenase mutant, a gene encoding the mutant, and a transformant, each enabling efficient production of crotyl alcohol, which serves as a raw material for producing butadiene; and a method for producing crotyl alcohol.

### Solution to Problem

The present inventor has conducted extensive investigations to solve the above-mentioned problems and has obtained the following findings.
(i) As described above, many bacteria inherently lack the ability to produce crotyl alcohol on an industrial scale. The crotyl alcohol-producing ability of bacteria is enhanced by introducing, into bacteria, a gene encoding a multifunctional enzyme having crotonaldehyde dehydrogenase activity, which converts crotonyl-CoA to crotonaldehyde, and crotyl alcohol dehydrogenase activity, which converts crotonaldehyde to crotyl alcohol. Hereinafter, this multifunctional enzyme is sometimes referred to as "crotonaldehyde-crotyl alcohol dehydrogenase", which is a type of aldehyde-alcohol dehydrogenase suitable for the production of crotyl alcohol.
(ii) A polypeptide consisting of the amino acid sequence of SEQ ID NO: 2 from *Fonticella tunisiensis* is an enzyme that has low crotonaldehyde-crotyl alcohol dehydrogenase activity. The function of this enzyme was first identified in the present invention, and there was no previous report on this function.
   Since this enzyme exhibits low activity, its activity needs to be enhanced for industrial-scale production of crotyl alcohol. Although mutagenesis is commonly used as a technique for enhancing enzyme activity, there is no information necessary to determine suitable mutation sites in this enzyme, including the substrate- or cofactor-binding site and binding mode of this enzyme.
(iii) In order to analyze information on the substrate binding of this enzyme, the present inventor compared the amino acid sequences among this enzyme, an aldehyde-alcohol dehydrogenase from *Escherichia coli* which does not use crotonyl-CoA as a substrate and whose active center was known from previous analysis of the three-dimensional structure of this protein, and a crotonaldehyde-crotyl alcohol dehydrogenase from *Clostridium acetobutylicum* strain ATCC 824, which was reported to use crotonyl-CoA as a substrate (Microb Cell Fact (2018) 17: 194). Specifically, the present inventor predicted the amino acid residues constituting the substrate-binding site of each enzyme and identified their differences based on the information about the active center of the aldehyde-alcohol dehydrogenase from *Escherichia coli*. Further analysis was made in light of the fact that these three enzymes exhibit different activity levels using crotonyl-CoA as a substrate. The analysis led to the prediction that isoleucine at position 403 (hereinafter sometimes referred to as "I403") in the aldehyde-alcohol dehydrogenase from *Fonticella tunisiensis* plays a crucial role in crotonaldehyde-crotyl alcohol dehydrogenase activity when crotonyl-CoA is used as a substrate.
(iv) In order to verify this prediction, the present inventor prepared mutants of the crotonaldehyde-crotyl alcohol dehydrogenase from *Fonticella tunisiensis*, in which I403 was substituted with each of other 19 amino acids.
   A wild-type crotonaldehyde-crotyl alcohol dehydrogenase from *Fonticella tunisiensis* and the prepared 19 mutant enzymes, each expressed in *Escherichia coli*, were compared in terms of the activity to convert crotonyl-CoA to crotyl alcohol. The results showed that crotyl alcohol-producing activity was increased three- to more than twenty-fold in 12 mutant enzymes, which were a serine mutant (I403S), a glycine mutant (I403G), a methionine mutant (I403M), an alanine mutant (I403A), a proline mutant (I403P), a glutamine mutant (I403Q), a phenylalanine mutant (I403F), a leucine mutant (I403L), a tryptophan mutant (I403W), a lysine mutant (I403K), a cysteine mutant (I403C), and a glutamic acid mutant (I403E), as compared with the wild-type enzyme. This verified that I403 plays a crucial role in crotonaldehyde-crotyl alcohol dehydrogenase activity.
(v) Among the above 12 mutants, genes encoding the eight mutants with the highest-activity, which were the serine mutant (I403S), the glycine mutant (I403G), the methionine mutant (I403M), the alanine mutant (I403A), the proline mutant (I403P), the glutamine mutant (I403Q), the phenylalanine mutant (I403F), and the leucine mutant (I403L), as well as a gene encoding the wild-type crotonaldehyde-crotyl alcohol dehydrogenase from *Fonticella tunisiensis*, were individually introduced into *Hydrogenophilus* bacteria to obtain the respective transformants. These transformants were cultured using carbon dioxide as a sole carbon source, and each mutant enzyme was expressed at a level comparable to that of the wild-type enzyme.
(vi) Crotonaldehyde-crotyl alcohol dehydrogenase activity was evaluated using disrupted cells of the transformant producing a wild-type crotonaldehyde-crotyl alcohol dehydrogenase from *Fonticella tunisiensis* and the above eight transformants each producing a different mutant crotonaldehyde-crotyl alcohol dehydrogenase. The activity was evaluated by quantifying crotyl alcohol produced from crotonyl-CoA as a substrate using gas chromatography. The results showed that the production of crotyl alcohol using the wild-type enzyme was below the detection limit, whereas the production of crotyl alcohol was detected for each of the eight mutant enzymes.

It is difficult to produce functional proteins in *Hydrogenophilus* bacteria by introduction of exogenous genes; however, the expression of functional proteins was achieved in *Hydrogenophilus* bacteria by using these mutant enzyme genes.

The present invention has been completed on the basis of the above findings and provides the following [1] to [7].
[1] A gene encoding a crotonaldehyde-crotyl alcohol dehydrogenase mutant described in the following (1) or (2).
   (1) A DNA comprising a nucleotide sequence having, in the nucleotide sequence of SEQ ID NO: 1, a nucleotide substitution resulting in a substitution of isoleucine at position 403 of SEQ ID NO: 2 encoded by SEQ ID NO: 1 with serine, glycine, methionine, alanine, proline, glutamine, phenylalanine, leucine, tryptophan, lysine, cysteine, or glutamic acid
   (2) A DNA encoding a polypeptide described in the following (2A) or (2B):
      (2A) a polypeptide comprising an amino acid sequence that has 90% or more identity to an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid), or
      (2B) a polypeptide comprising an amino acid sequence that has 1 to 80 amino acids deleted, substituted, inserted, or added in an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid)
[2] The gene according to [1], wherein (1a) the above (1) is a DNA comprising the following nucleotide sequence:
   a nucleotide sequence having a substitution of ATA at positions 1207 to 1209 of SEQ ID NO: 1 with TCT, TCC, TCA, TCG, AGT, or AGC,
   a nucleotide sequence having a substitution of ATA at positions 1207 to 1209 of SEQ ID NO: 1 with GGT, GGC, GGA, or GGG,
   a nucleotide sequence having a substitution of ATA at positions 1207 to 1209 of SEQ ID NO: 1 with ATG,
   a nucleotide sequence having a substitution of ATA at positions 1207 to 1209 of SEQ ID NO: 1 with GCT, GCC, GCA, or GCG,
   a nucleotide sequence having a substitution of ATA at positions 1207 to 1209 of SEQ ID NO: 1 with CCT, CCC, CCA, or CCG,
   a nucleotide sequence having a substitution of ATA at positions 1207 to 1209 of SEQ ID NO: 1 with CAA or CAG,
   a nucleotide sequence having a substitution of ATA at positions 1207 to 1209 of SEQ ID NO: 1 with TTT or TTC,
   a nucleotide sequence having a substitution of ATA at positions 1207 to 1209 of SEQ ID NO: 1 with TTA, TTG, CTT, CTC, CTA, or CTG,
   a nucleotide sequence having a substitution of ATA at positions 1207 to 1209 of SEQ ID NO: 1 with TGG,
   a nucleotide sequence having a substitution of ATA at positions 1207 to 1209 of SEQ ID NO: 1 with AAA or AAG,
   a nucleotide sequence having a substitution of ATA at positions 1207 to 1209 of SEQ ID NO: 1 with TGT or TGC, or
   a nucleotide sequence having a substitution of ATA at positions 1207 to 1209 of SEQ ID NO: 1 with GAA or GAG, and
   wherein the above (2) is a DNA comprising a nucleotide sequence that has 90% or more identity to any one of the nucleotide sequences described in the above (1a) and encoding a polypeptide having crotonaldehyde-crotyl alcohol dehydrogenase activity (wherein the nucleotide sequence of the DNA has a deletion, substitution, insertion, or addition of a nucleotide or nucleotides at a position or positions other than positions 1207 to 1209 of the corresponding nucleotide sequence in the above (1a)).
[3] The gene according to [1] or [2], wherein the gene is a DNA described in the following (A) or (B).
   (A) A DNA comprising any one of the nucleotide sequences of SEQ ID NOS: 3 to 14
   (B) A DNA comprising a nucleotide sequence that has 90% or more identity to SEQ ID NOS: 3 to 14 and encoding a polypeptide having crotonaldehyde-crotyl alcohol dehydrogenase activity (wherein the nucleotide sequence of the DNA has a deletion, substitution, insertion, or addition of a nucleotide or nucleotides at a position or positions other than positions 1207 to 1209 of SEQ ID NOS: 3 to 10)
[4] A crotonaldehyde-crotyl alcohol dehydrogenase mutant described in the following (3) or (4).
   (3) A polypeptide comprising an amino acid sequence that has a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2
   (4) A polypeptide described in the following (4A) or (4B):
      (4A) a polypeptide comprising an amino acid sequence that has 90% or more identity to an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid), or
      (4B) a polypeptide comprising an amino acid sequence that has 1 to 80 amino acids deleted, substituted, inserted, or added in an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid)
   [5] A transformant obtained by introducing the gene according to any one of [1] to [3] into a bacterium.
   [6] The transformant according to [5], wherein the bacterium is a hydrogen-oxidizing bacterium.
   [7] A method for producing crotyl alcohol, the method comprising a step of culturing the transformant according to [5] or [6].

### Advantageous Effects of Invention

The recently reported four-step continuous process for butadiene production is a method in which butadiene is produced from ethanol via acetaldehyde, crotonaldehyde, and crotyl alcohol. Since each step from ethanol to butadiene is carried out by chemical synthesis, the use of bio-ethanol alone does not sufficiently reduce the environmental burden. Therefore, all or part of this process needs to be carried out by bioprocessing.

The crotonaldehyde-crotyl alcohol dehydrogenase mutant of the present invention produces crotyl alcohol with high efficiency. Generally, bacteria do not produce crotyl alcohol on an industrial scale; however, by transforming bacteria with a gene encoding the crotonaldehyde-crotyl alcohol dehydrogenase mutant of the present invention and culturing the resulting transformant, crotyl alcohol can be produced efficiently with a reduced environmental burden. When the thus-obtained bio-crotyl alcohol is converted into butadiene in a one-step reaction, the use of chemical raw materials can be reduced, thereby improving material sustainability. Furthermore, bio-butadiene can be produced from bio-crotyl alcohol using microorganisms.

Alternatively, by in vitro reaction of the crotonaldehyde-crotyl alcohol dehydrogenase mutant of the present invention with crotonyl-CoA, crotyl alcohol can be efficiently produced with a reduced environmental burden.

Measures to counter the increase in atmospheric carbon dioxide entail reduction of carbon dioxide emissions and fixation of emitted carbon dioxide. In order to reduce carbon dioxide emissions, solar, wind, geothermal, and similar energies are utilized in place of fossil energy. However, the utilization of such energies is not yet extensive enough to repress the buildup of atmospheric carbon dioxide. Consequently, there is need to enhance atmospheric carbon fixation or recycling of emitted carbon dioxide.

Carbon dioxide can be fixed through physical or chemical processes. However, fixation of carbon dioxide utilizing living cells allows the production of organic substances that can be used as food, feed, or fuel. In other words, carbon dioxide itself can be directly converted into valuable substances. Accordingly, the twin problems of global warming due to increased atmospheric carbon dioxide and scarcity of food, feed, and fuel can be solved. Further, in-demand chemical product raw materials can be produced while suppressing global warming attributed to increased carbon dioxide emissions.

Hydrogen-oxidizing bacteria that can grow by utilizing the chemical energy generated by the reaction of hydrogen with oxygen and by using carbon dioxide as a sole carbon source, can produce chemical products from a mixture of oxygen, hydrogen, and carbon dioxide gases as raw material. Therefore, the bacteria can achieve the efficient conversion of carbon dioxide into organic compounds and can be cultured in a simple culture medium. Typically, hydrogen-oxidizing bacteria grow slowly, but the growth rate of *Hydrogenophilus* bacteria among hydrogen-oxidizing bacteria is exceptionally high. The Journal of Mitsubishi Research Institute No. 34 1999 describes *Hydrogenophilus* bacteria as follows: "Their proliferative capacity is so high that their carbon dioxide fixation ability cannot be compared with that of plants, which truly indicates the high carbon dioxide fixation ability of microorganisms".

*Hydrogenophilus* bacteria inherently have genes encoding enzymes that catalyze the conversion of pyruvate to crotonyl-CoA, but lack a gene encoding an enzyme that catalyzes the conversion of crotonyl-CoA to crotyl alcohol.

The present inventor has found that it is difficult to express functional proteins in *Hydrogenophilus* bacteria by introduction of exogenous genes; however, a gene encoding crotonaldehyde-crotyl alcohol dehydrogenase mutant of the present invention can be expressed in *Hydrogenophilus* bacteria, yielding an enzyme that functions as crotonaldehyde-crotyl alcohol dehydrogenase. Therefore, a transformant obtained by introducing the mutant gene of the present invention into *Hydrogenophilus* bacteria can grow rapidly using carbon dioxide as a sole carbon source and produces bio-crotyl alcohol with high efficiency.

The mutant of the present invention enables efficient production of crotyl alcohol than the wild-type enzyme. In particular, when *Hydrogenophilus* bacteria are used as hosts, the production rate of crotyl alcohol drastically increases. Furthermore, since crotyl alcohol can be produced through carbon dioxide fixation, industrial production of crotyl alcohol can be achieved with a reduced environmental burden.

### Brief Description of Drawings

[Fig. 1] Fig. 1 illustrates a metabolic pathway found in many microorganisms for converting pyruvate to butadiene via crotonyl-CoA.
[Fig. 2] Fig. 2 shows the results of a comparison between a wild-type crotonaldehyde-crotyl alcohol dehydrogenase from *Fonticella tunisiensis* and prepared 19 mutant enzymes, each expressed in *Escherichia coli*, in terms of the activity to convert crotonyl-CoA to crotyl alcohol. The relative values of the crotyl alcohol peak areas obtained from the mutant enzymes are shown, with the crotyl alcohol peak area obtained from the wild-type enzyme set to 1.

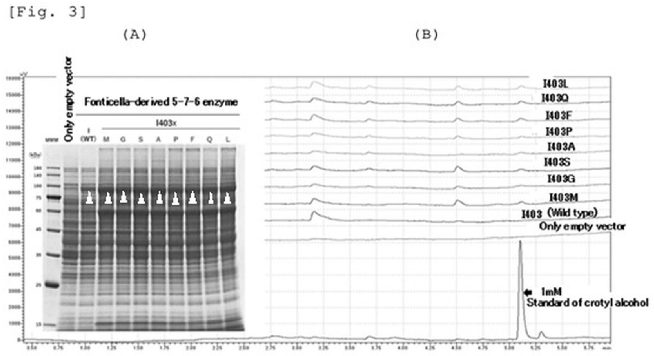

[Fig. 3] Fig. 3(A) presents a gel image showing the results of polyacrylamide gel electrophoresis of proteins contained in cultures of transformants obtained by introducing, into *Hydrogenophilus thermoluteolus*, a gene encoding a wild-type crotonaldehyde-crotyl alcohol dehydrogenase from *Fonticella tunisiensi*s and genes encoding eight mutant enzymes. Fig. 3(B) presents gas chromatography chromatograms showing that crotyl alcohol was produced from crotonyl-CoA as a substrate by the crotonaldehyde-crotyl alcohol dehydrogenase mutants produced by *Hydrogenophilus thermoluteolus*.

### Description of Embodiments

The present invention will be described in detail below.

### (1) Crotonaldehyde-crotyl alcohol dehydrogenase mutant

The crotonaldehyde-crotyl alcohol dehydrogenase mutant of the present invention is a polypeptide comprising an amino acid sequence having a substitution of isoleucine (Ile) at position 403 with serine (Ser), glycine (Gly), methionine (Met), alanine (Ala), proline (Pro), glutamine (Gln), phenylalanine (Phe), leucine (Leu), tryptophan (Trp), lysine (Lys), cysteine (Cys), or glutamic acid (Glu) in the amino acid sequence of SEQ ID NO: 2 (in particular, a polypeptide consisting of an amino acid sequence having a substitution of Ile at position 403 with Ser, Gly, Met, Ala, Pro, Gln, Phe, Leu, Trp, Lys, Cys, or Glu in the amino acid sequence of SEQ ID NO: 2) or a homolog of the polypeptide. In the homolog, the amino acid at a position equivalent or corresponding to position 403 of SEQ ID NO: 2 is Ser, Gly, Met, Ala, Pro, Gln, Phe, Leu, Trp, Lys, Cys, or Glu.

SEQ ID NO: 2 represents the amino acid sequence of a wild-type crotonaldehyde-crotyl alcohol dehydrogenase from *Fonticella tunisiensis*.

The homolog may be a polypeptide comprising an amino acid sequence that has 90% or more, even 95% or more, even 98% or more, or even 99% or more identity to an amino acid sequence having a substitution of Ile at position 403 with Ser, Gly, Met, Ala, Pro, Gln, Phe, Leu, Trp, Lys, Cys, or Glu in the amino acid sequence of SEQ ID NO: 2 (in particular, a polypeptide consisting of an amino acid sequence that has 90% or more, even 95% or more, even 98% or more, or even 99% or more identity to an amino acid sequence having a substitution of Ile at position 403 with Ser, Gly, Met, Ala, Pro, Gln, Phe, Leu, Trp, Lys, Cys, or Glu in the amino acid sequence of SEQ ID NO: 2) and having crotonaldehyde-crotyl alcohol dehydrogenase activity.

The homolog may also be a polypeptide comprising an amino acid sequence that has 1 to 80, 1 to 50, 1 to 30, 1 to 10, 1 to 5, 1 to 4, 1 to 3, or 1 to 2 amino acids, or 1 amino acid deleted, substituted, inserted, or added in an amino acid sequence having a substitution of Ile at position 403 with Ser, Gly, Met, Ala, Pro, Gln, Phe, Leu, Trp, Lys, Cys, or Glu in the amino acid sequence of SEQ ID NO: 2 (in particular, a polypeptide consisting of an amino acid sequence that has 1 to 80, 1 to 50, 1 to 30, 1 to 10, 1 to 5, 1 to 4, 1 to 3, or 1 to 2 amino acids, or 1 amino acid deleted, substituted, inserted, or added in an amino acid sequence having a substitution of Ile at position 403 with Ser, Gly, Met, Ala, Pro, Gln, Phe, Leu, Trp, Lys, Cys, or Glu in the amino acid sequence of SEQ ID NO: 2) and having crotonaldehyde-crotyl alcohol dehydrogenase activity.

In these homologs, the amino acid at a position equivalent or corresponding to position 403 of SEQ ID NO: 2 is Ser, Gly, Met, Ala, Pro, Gln, Phe, Leu, Trp, Lys, Cys, or Glu. That is, these homologs have, in an amino acid sequence having a substitution of Ile at position 403 of SEQ ID NO: 2 with Ser, Gly, Met, Ala, Pro, Gln, Phe, Leu, Trp, Lys, Cys, or Glu, a modification (deletion, substitution, insertion, or addition) of an amino acid residue or amino acid residues other than the amino acid residue subjected to that substitution.

In the present invention, the presence of crotonaldehyde dehydrogenase activity using crotonyl-CoA as a substrate and crotyl alcohol dehydrogenase activity using crotonaldehyde as a substrate in a polypeptide of interest is determined by reacting the polypeptide with crotonyl-CoA in the presence of NADH and detecting the resulting crotyl alcohol by gas chromatography.

### (2) Crotonaldehyde-crotyl alcohol dehydrogenase gene mutant

The gene encoding the crotonaldehyde-crotyl alcohol dehydrogenase mutant of the present invention is a DNA comprising a nucleotide sequence having, in the nucleotide sequence of SEQ ID NO: 1, a nucleotide substitution resulting in a substitution of Ile at position 403 of SEQ ID NO: 2 encoded by SEQ ID NO: 1 with Ser, Gly, Met, Ala, Pro, Gln, Phe, Leu, Trp, Lys, Cys, or Glu (in particular, a DNA consisting of a nucleotide sequence having, in the nucleotide sequence of SEQ ID NO: 1, a nucleotide substitution resulting in a substitution of Ile at position 403 of SEQ ID NO: 2 encoded by SEQ ID NO: 1 with Ser, Gly, Met, Ala, Pro, Gln, Phe, Leu, Trp, Lys, Cys, or Glu) or a homologous DNA thereof. In the homologous DNA, the trinucleotide encoding the amino acid at a position equivalent or corresponding to position 403 of SEQ ID NO: 2 in the amino acid sequence encoded by the homologous DNA is a trinucleotide encoding Ser, Gly, Met, Ala, Pro, Gln, Phe, Leu, Trp, Lys, Cys, or Glu.

SEQ ID NO: 1 represents a DNA encoding a wild-type crotonaldehyde-crotyl alcohol dehydrogenase from *Fonticella tunisiensis*.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Ser may be a nucleotide sequence having a substitution of ATA at positions 1207 to 1209 of SEQ ID NO: 1 with TCT, TCC, TCA, TCG, AGT, or AGC (this is sometimes referred to as "nucleotide sequence I403S"). In particular, a nucleotide sequence having a substitution of ATA at positions 1207 to 1209 of SEQ ID NO: 1 with TCG (SEQ ID NO: 3) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Gly may be a nucleotide sequence having a substitution of ATA at positions 1207 to 1209 of SEQ ID NO: 1 with GGT, GGC, GGA, or GGG (this is sometimes referred to as "nucleotide sequence I403G"). In particular, a nucleotide sequence having a substitution of ATA at positions 1207 to 1209 of SEQ ID NO: 1 with GGC (SEQ ID NO: 4) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Met may be a nucleotide sequence having a substitution of ATA at positions 1207 to 1209 of SEQ ID NO: 1 with ATG (this is sometimes referred to as "nucleotide sequence I403M"). In particular, the nucleotide sequence having a substitution of ATA at positions 1207 to 1209 of SEQ ID NO: 1 with ATG (SEQ ID NO: 5) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Ala may be a nucleotide sequence having a substitution of ATA at positions 1207 to 1209 of SEQ ID NO: 1 with GCT, GCC, GCA, or GCG (this is sometimes referred to as "nucleotide sequence I403A"). In particular, a nucleotide sequence having a substitution of ATA at positions 1207 to 1209 of SEQ ID NO: 1 with GCG (SEQ ID NO: 6) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Pro may be a nucleotide sequence having a substitution of ATA at positions 1207 to 1209 of SEQ ID NO: 1 with CCT, CCC, CCA, or CCG (this is sometimes referred to as "nucleotide sequence I403P"). In particular, a nucleotide sequence having a substitution of ATA at positions 1207 to 1209 of SEQ ID NO: 1 with CCG (SEQ ID NO: 7) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Gln may be a nucleotide sequence having a substitution of ATA at positions 1207 to 1209 of SEQ ID NO: 1 with CAA or CAG (this is sometimes referred to as "nucleotide sequence I403Q"). In particular, a nucleotide sequence having a substitution of ATA at positions 1207 to 1209 of SEQ ID NO: 1 with CAG (SEQ ID NO: 8) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Phe may be a nucleotide sequence having a substitution of ATA at positions 1207 to 1209 of SEQ ID NO: 1 with TTT or TTC (this is sometimes referred to as "nucleotide sequence I403F"). In particular, a nucleotide sequence having a substitution of ATA at positions 1207 to 1209 of SEQ ID NO: 1 with TTC (SEQ ID NO: 9) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Leu may be a nucleotide sequence having a substitution of ATA at positions 1207 to 1209 of SEQ ID NO: 1 with TTA, TTG, CTT, CTC, CTA, or CTG (this is sometimes referred to as "nucleotide sequence I403L"). In particular, a nucleotide sequence having a substitution of ATA at positions 1207 to 1209 of SEQ ID NO: 1 with CTC (SEQ ID NO: 10) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Trp may be a nucleotide sequence having a substitution of ATA at positions 1207 to 1209 of SEQ ID NO: 1 with TGG (this is sometimes referred to as "nucleotide sequence I403W"). In particular, the nucleotide sequence having a substitution of ATA at positions 1207 to 1209 of SEQ ID NO: 1 with TGG (SEQ ID NO: 11) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Lys may be a nucleotide sequence having a substitution of ATA at positions 1207 to 1209 of SEQ ID NO: 1 with AAA or AAG (this is sometimes referred to as "nucleotide sequence I403K"). In particular, a nucleotide sequence having a substitution of ATA at positions 1207 to 1209 of SEQ ID NO: 1 with AAA (SEQ ID NO: 12) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Cys may be a nucleotide sequence having a substitution of ATA at positions 1207 to 1209 of SEQ ID NO: 1 with TGT or TGC (this is sometimes referred to as "nucleotide sequence I403C"). In particular, a nucleotide sequence having a substitution of ATA at positions 1207 to 1209 of SEQ ID NO: 1 with TGC (SEQ ID NO: 13) is preferable.

The nucleotide sequence having, in the nucleotide sequence of SEQ ID NO: 1, a nucleotide substitution resulting in the substitution of Ile at position 403 of SEQ ID NO: 2 encoded by SEQ ID NO: 1 with Glu may be a nucleotide sequence having a substitution of ATA at positions 1207 to 1209 of SEQ ID NO: 1 with GAA or GAG (this is sometimes referred to as "nucleotide sequence I403E"). In particular, a nucleotide sequence having a substitution of ATA at positions 1207 to 1209 of SEQ ID NO: 1 with GAA (SEQ ID NO: 14) is preferable.

The homologous DNA is a DNA encoding a homolog of the crotonaldehyde-crotyl alcohol dehydrogenase mutant of the present invention described above.

The homologous DNA may be a DNA comprising a nucleotide sequence that has 90% or more, even 95% or more, even 98% or more, or even 99% or more identity to the nucleotide sequence I403S, the nucleotide sequence I403G, the nucleotide sequence I403M, the nucleotide sequence I403A, the nucleotide sequence I403P, the nucleotide sequence I403Q, the nucleotide sequence I403F, the nucleotide sequence I403L, the nucleotide sequence I403W, the nucleotide sequence I403K, the nucleotide sequence I403C, or the nucleotide sequence I403E (in particular, a DNA consisting of a nucleotide sequence that has 90% or more, even 95% or more, even 98% or more, or even 99% or more identity to the nucleotide sequence I403S, the nucleotide sequence I403G, the nucleotide sequence I403M, the nucleotide sequence I403A, the nucleotide sequence I403P, the nucleotide sequence I403Q, the nucleotide sequence I403F, the nucleotide sequence I403L, the nucleotide sequence I403W, the nucleotide sequence I403K, the nucleotide sequence I403C, or the nucleotide sequence I403E) and encoding a polypeptide having crotonaldehyde-crotyl alcohol dehydrogenase activity.

The homologous DNA comprises a nucleotide sequence having a modification (deletion, substitution, insertion, or addition) of a nucleotide or nucleotides at a position or positions other than positions 1207 to 1209 of the nucleotide sequence I403S, the nucleotide sequence I403G, the nucleotide sequence I403M, the nucleotide sequence I403A, the nucleotide sequence I403P, the nucleotide sequence I403Q, the nucleotide sequence I403F, the nucleotide sequence I403L, the nucleotide sequence I403W, the nucleotide sequence I403K, the nucleotide sequence I403C, or the nucleotide sequence I403E.

In the present invention, the identities of nucleotide sequences and amino acid sequences are calculated using GENETYX version 17 (GENETYX Corporation).

The crotonaldehyde-crotyl alcohol dehydrogenase mutant of the present invention and the gene encoding the crotonaldehyde-crotyl alcohol dehydrogenase mutant of the present invention may be naturally occurring in the environment, for example, resulting from spontaneous mutations; artificially synthesized using methods known to those skilled in the art; obtained by site-directed mutagenesis of the native DNA using PCR techniques; or obtained by mutagen treatment.

### (2) Transformant capable of producing crotyl alcohol

The transformant (transformed cell) of the present invention is a transformant obtained by introducing a gene encoding the crotonaldehyde-crotyl alcohol dehydrogenase mutant of the present invention into a host bacterium. In other words, the transformant of the present invention has an exogenous gene encoding the crotonaldehyde-crotyl alcohol dehydrogenase mutant of the present invention.

The transformant of the present invention may be a transformant obtained by introduction of one or more crotonaldehyde-crotyl alcohol dehydrogenase gene mutants of the present invention. The transformant of the present invention may also be a transformant obtained by introduction of not only a crotonaldehyde-crotyl alcohol dehydrogenase gene mutant of the present invention, but also another gene or other genes.

### Host bacteria

Examples of host bacteria include, but are not limited to, *Escherichia coli*, *Corynebacterium glutamicum*, *Bacillus subtilis*, and *Clostridium thermocellum*.

When hydrogen-oxidizing bacteria are used as hosts, crotyl alcohol can be produced using carbon dioxide as a sole carbon source.

Examples of the hydrogen-oxidizing bacteria include *Pseudonocardia* bacteria such as *Pseudonocardia autotrophica*; *Hydrogenobacter* bacteria such as *Hydrogenobacter hydrogenophilus*; *Hydrogenophilus* bacteria such as *Hydrogenophilus thermoluteolus*, *Hydrogenophilus thiooxidans*, *Hydrogenophilus halorhabdus*, *Hydrogenophilus denitrificans*, *Hydrogenophilus hirschii*, *Hydrogenophilus islandicus*, *Hydrogenophilu*s sp. Mar3, *Hydrogenophilus* sp. Z1038; *Paracoccus* bacteria such as *Paracoccus denitrificans*, *Paracoccus pantotrophus*; *Xanthobacter* bacteria such as *Xanthobacter autotrophicus*; *Cupriavidu*s bacteria such as *Cupriavidus basilensis*, *Cupriavidus metallidurans*, *Cupriavidus necator*, *Cupriavidus pauculus*, *Cupriavidus taiwanensis*; *Hydrogenophaga* bacteria such as *Hydrogenophaga pseudoflava*; and *Variovorax* bacteria such as *Variovorax paradoxus*.

Among these bacteria, *Hydrogenophilus* bacteria are preferable, *Hydrogenophilus thermoluteolus* is more preferable because they have top-level growth rate, and therefore, top-level carbon dioxide fixation ability among carbon dioxide fixing microorganisms.

*Hydrogenophilus* bacteria can be easily isolated from diverse regions everywhere on the earth. Preferable strains of *Hydrogenophilus thermoluteolus* include strain TH-1 (NBRC 14978). H*ydrogenophilus thermoluteolus* strain TH-1 (NBRC 14978) exhibits the highest rapid growth rate among carbon dioxide fixing microorganisms (Agricultural and Biological Chemistry, 41, 685-690 (1977))(It proliferates double per hour.). H*ydrogenophilus thermoluteolus* strain NBRC 14978 is internationally deposited under the Budapest Treaty, and is thus publicly available.

Host bacteria may be bacteria isolated from nature or genetically modified bacteria derived from bacteria isolated from nature. Genetic modification may be intended, for example, to allow high expression of an introduced gene.

Such a genetic modification can be made, for example, by removing (curing) endogenous plasmids in bacteria, or disrupting genes on the genome of bacteria. Methods for removing endogenous plasmids are well known and include, for example, treating with chemicals such as novobiocin, SDS, acriflavine, and ethidium bromide; methods utilizing the phenomenon of plasmid incompatibility such as a method of introducing a plasmid having the same origin of replication as those of endogenous plasmids to destabilize the endogenous plasmids, and a method of disrupting factors involved in the plasmid partition system to destabilize endogenous plasmids.

### Methods for producing transformants

Methods for obtaining transformants by introducing a mutant gene of the present invention into a host bacterium are described below.

The introduction of a mutant gene into a host bacterium can be carried out using common methods for introducing exogenous genes into bacteria. These genes may be directly introduced into host bacteria. Alternatively, they may be incorporated into a vector for transformation such as a plasmid vector, viral vector, cosmid, fosmid, or BAC, and then, an obtained vector may be introduced into host bacteria.

Vectors for transformation should contain a DNA which controls the autonomous replication function within host bacteria, and examples include broad-host-range vectors pRK415 (GenBank: EF437940.1), pBHR1 (GenBank: Y14439.1), pMMB67EH (ATCC 37622), pCAR1 (NCBI Reference Sequence: NC_004444.1), pC194 (NCBI Reference Sequence: NC_002013.1), pK18mobsacB (GenBank: FJ437239.1), pUB110 (NCBI Reference Sequence: NC_001384.1), vectors obtained by genetically modifying these vectors (For example, pCAMO-6), and the like.

Among them, pCAMO-6 is preferable. Those skilled in the art can prepare pCAMO-6 based on its nucleotide sequence (SEQ ID NO:15).

Examples of promoters in vectors include tac promoter, lac promoter, trc promoter, or each of promoters OXB1 and OXB11 to OXB20 from Oxford Genetics Ltd. Examples of terminators in vectors include the T1T2 terminator of *Escherichia coli* rRNA operon rrnB, the t0 transcription terminator of bacteriophage λ, T7 terminator, and the like.

Introduction of these genes into a host bacterium (transformation) can be carried out by publicly known methods such as calcium chloride method, rubidium chloride method, and electric pulse method (electroporation method).

### (2) Method for producing crotyl alcohol

The present invention provides a method for producing crotyl alcohol with use of the transformant of the present invention described above. The method includes a step of culturing the transformant of the present invention. Particularly, the method includes a step of culturing the transformant of the present invention using an inorganic or organic culture medium while supplying a gas containing carbon dioxide, preferably, a mixture of gases containing hydrogen, oxygen, and carbon dioxide. The supplied gas is preferably a mixture of gases consisting of hydrogen, oxygen, and carbon dioxide. However, different kinds of gases can be mixed within, to the extent that crotyl alcohol can be produced efficiently.

*Hydrogenophilus* bacteria can grow using hydrogen as a source of energy and using carbon dioxide as a sole carbon source, and thus, carbon dioxide can be fixed efficiently particularly by producing crotyl alcohol by using substantially only carbon dioxide (in particular, by using only carbon dioxide) as a carbon source. Therefore, using an inorganic culture medium that does not contain carbon sources such as organic substances and carbonates is preferable. Namely, carrying out culture using substantially only carbon dioxide (in particular, using only carbon dioxide) as a carbon source is preferable. "Using substantially only carbon dioxide as a carbon source" encompass cases in which an unavoidable amount of other carbon sources is mixed within.

The pH of the culture medium is preferably 6.2 to 8, more preferably 6.4 to 7.4, and furthermore preferably 6.6 to 7. When the pH is within this range, bacteria grow well and mixed gases dissolves well into the culture medium, and crotyl alcohol can be produced efficiently.

When batch culture is utilized, mixed gases can be entrapped within an airtight culture container and static culture or shaking culture can be carried out. Shaking culture is preferable in that better dissolution of mixed gases into the culture medium can be achieved. When continuous culture is utilized, mixed gases can be continuously supplied into a culture container and shaking culture can be carried out, or the transformant can be cultured while introducing mixed gases into the culture medium by bubbling and mixing the culture medium.

The volume ratio of hydrogen, oxygen, and carbon dioxide (hydrogen: oxygen: carbon dioxide) in the supplied gas mixture is preferably 1.75 to 7.5:1:0.25 to 3, more preferably 5 to 7.5:1:1 to 2, and even more preferably 6.25 to 7.5:1:1.5. When the volume ratio is within this range, bacteria grow well, and crotyl alcohol can be produced efficiently.

The supply rate of mixed gases or raw material gases can be 10 to 60 L/hour, in particular 10 to 40 L/hour, in particular 10 to 20 L/hour, per 1 L of culture medium. When the supply rate is within this range, transformants grow well and crotyl alcohol can be produced efficiently, and the amount of wasted mixed gases can be reduced.

The culture temperature is preferably 35 to 55°C, more preferably 37 to 52°C, and even more preferably 50 to 52°C. When the temperature is within this range, transformants grow well, and crotyl alcohol can be produced efficiently.

Crotyl alcohol is produced in the medium solution by culturing the transformant in the above-described manner. Collecting the medium solution will enable the recovery of crotyl alcohol, however, crotyl alcohol can furthermore be separated from the medium solution by publicly known methods. Such publicly known methods include membrane separation, distillation, and the like.

### Examples

The present invention will now be described with reference to examples. The technical scope of the present invention is not limited by the following examples.

### (1) Construction of gene expression plasmids

Gene expression plasmids were constructed by Gibson Assembly.

First, a DNA fragment consisting of a crotonaldehyde-crotyl alcohol dehydrogenase gene that consists of the nucleotide sequence of SEQ ID NO: 1 was amplified by PCR using the genomic DNA of *Fonticella tunisiensis* as a template. The primers shown below were used for PCR.
(a-1) 5'-GATCTGGAGGAGAAACGCATATGAAAGTCACCAACGCGGAAG-3' (SEQ ID NO: 16)
(a-2) 5'-TGTCGACGGAGCTCGAATTCTCATTTGTTGTTTTCTTCGGTTTC-3' (SEQ ID NO: 17)

The primers (a-1) and (a-2) contain sequences homologous to regions of vector pCAMO-6.

The resulting reaction mixture was subjected to agarose gel electrophoresis. The results showed that an approximately 2.7-kbp DNA fragment was detected. A gel portion containing this DNA fragment was cut out of the agarose gel, and the DNA fragment was recovered from the gel portion with GEL/PCR Purification Mini Kit (FAVORGEN).

The pCAMO-6 plasmid vector was amplified by PCR using a DNA consisting of the nucleotide sequence of SEQ ID NO:15 for DNA ligation by Gibson Assembly. The primers shown below were used for PCR.

### Primers for amplification of pCAMO-6 plasmid vector

| | | |
|---|---|---|
| (a-20) | 5'-GAATTCGAGCTCCGTCGACA-3' | (SEQ ID NO: 18) |
| (b-20) | 5'-ATGCGTTTCTCCTCCAGATC-3' | (SEQ ID NO: 19) |

The resulting reaction mixture was subjected to electrophoresis on an agarose gel. The results showed that about 5.5-kbp DNA fragment was detected. The gel portion containing the vector DNA fragment was cut out of the agarose gel, and the vector DNA fragment was collected from the gel portion with GEL/PCR Purification Mini Kit (FAVORGEN).

The DNA fragment containing the pCAMO-6 vector as synthesized above and the DNA fragment containing the crotonaldehyde-crotyl alcohol dehydrogenase gene were joined together with Gibson Assembly Master Mix manufactured by New England Biolabs Inc.

With each of the resulting reaction mixtures, *Escherichia coli* JM109 strain was transformed by heat shock method, applied to LB media containing 50 µg/mL kanamycin, and cultured at 37°C for 24 hours.

Each of the strains grown on the LB media was inoculated with a platinum loop into 5 mL of liquid LB medium containing 50 µg/mL kanamycin in a test tube, and cultured with shaking at 37°C. Plasmid DNA was extracted from the culture liquid (pCAMO-6-Fonti). The sequence of the gene inserted in the plasmid was analyzed by the Sanger method by Eurofins Genomics K.K. under contract, and confirmed to be identical to the corresponding sequence in a database.

The constructed plasmid can be replicated in *Hydrogenophilus thermoluteolus* (TH-1) NBRC 14978 strain as well as in *Escherichia coli*.

Tables 1A and 1B show the primers used to mutate isoleucine at position 403 (I403) of the wild-type crotonaldehyde-crotyl alcohol dehydrogenase from *Fonticella tunisensis* to each of other 19 amino acids by using, as a template, the constructed plasmid (pCAMO-6-Fonti) containing the gene encoding the enzyme.

**[Table 1A]**

| Amino acid^{*} | Primer name | Primer sequence (5'-3') |
|---|---|---|
| Ser | I403S-1 | TCGTCGCAGGGCGCGTCGGGCGACATCTACAAC (SEQ ID NO: 20) |
| | I403S-2 | GTTGTAGATGTCGCCCGACGCGCCCTGCGACGA (SEQ ID NO: 21) |
| Gly | I403G-1 | TCGTCGCAGGGCGCGGGCGGCGACATCTACAAC (SEQ ID NO: 22) |
| | I403G-2 | GTTGTAGATGTCGCCGCCCGCGCCCTGCGACGA (SEQ ID NO: 23) |
| Ala | I403A-1 | TCGTCGCAGGGCGCGGCGGGCGACATCTACAAC (SEQ ID NO: 24) |
| | I403A-2 | GTTGTAGATGTCGCCCGCCGCGCCCTGCGACGA (SEQ ID NO: 25) |
| Phe | I403F-1 | TCGTCGCAGGGCGCGTTCGGCGACATCTACAAC (SEQ ID NO: 26) |
| | I403F-2 | GTTGTAGATGTCGCCGAACGCGCCCTGCGACGA (SEQ ID NO: 27) |
| Leu | I403L-1 | TCGTCGCAGGGCGCGCTCGGCGACATCTACAAC (SEQ ID NO: 28) |
| | I403L-2 | GTTGTAGATGTCGCCGAGCGCGCCCTGCGACGA (SEQ ID NO: 29) |
| Met | I403M-1 | TCGTCGCAGGGCGCGATGGGCGACATCTACAAC (SEQ ID NO: 30) |
| | I403M-2 | GTTGTAGATGTCGCCCATCGCGCCCTGCGACGA (SEQ ID NO: 31) |
| Val | I403V-1 | TCGTCGCAGGGCGCGGTCGGCGACATCTACAAC (SEQ ID NO: 32) |
| | I403V-2 | GTTGTAGATGTCGCCGACCGCGCCCTGCGACGA (SEQ ID NO: 33) |
| Pro | I403P-1 | TCGTCGCAGGGCGCGCCGGGCGACATCTACAAC (SEQ ID NO: 34) |
| | I403P-2 | GTTGTAGATGTCGCCCGGCGCGCCCTGCGACGA (SEQ ID NO: 35) |
| Thr | I403T-1 | TCGTCGCAGGGCGCGACCGGCGACATCTACAAC (SEQ ID NO: 36) |
| | I403T-2 | GTTGTAGATGTCGCCGGTCGCGCCCTGCGACGA (SEQ ID NO: 37) |
| Tyr | I403Y-1 | TCGTCGCAGGGCGCGTACGGCGACATCTACAAC (SEQ ID NO: 38) |
| | I403Y-2 | GTTGTAGATGTCGCCGTACGCGCCCTGCGACGA (SEQ ID NO: 39) |

**[Table 1B]**

| Amino acid^{*} | Primer name | Primer sequence (5'-3') |
|---|---|---|
| His | I403H-1 | TCGTCGCAGGGCGCGCACGGCGACATCTACAAC (SEQ ID NO: 40) |
| | I403H-2 | GTTGTAGATGTCGCCGTGCGCGCCCTGCGACGA (SEQ ID NO: 41) |
| Asn | I403N-1 | TCGTCGCAGGGCGCGAACGGCGACATCTACAAC (SEQ ID NO: 42) |
| | I403N-2 | GTTGTAGATGTCGCCGTTCGCGCCCTGCGACGA (SEQ ID NO: 43) |
| Gln | I403Q-1 | TCGTCGCAGGGCGCGCAGGGCGACATCTACAAC (SEQ ID NO: 44) |
| | I403Q-2 | GTTGTAGATGTCGCCCTGCGCGCCCTGCGACGA (SEQ ID NO: 45) |
| Lys | I403K-1 | TCGTCGCAGGGCGCGAAAGGCGACATCTACAAC (SEQ ID NO: 46) |
| | I403K-2 | GTTGTAGATGTCGCCTTTCGCGCCCTGCGACGA (SEQ ID NO: 47) |
| Asp | I403D-1 | TCGTCGCAGGGCGCGGACGGCGACATCTACAAC (SEQ ID NO: 48) |
| | I403D-2 | GTTGTAGATGTCGCCGTCCGCGCCCTGCGACGA (SEQ ID NO: 49) |
| Glu | I403E-1 | TCGTCGCAGGGCGCGGAAGGCGACATCTACAAC (SEQ ID NO: 50) |
| | I403E-2 | GTTGTAGATGTCGCCTTCCGCGCCCTGCGACGA (SEQ ID NO: 51) |
| Cys | I403C-1 | TCGTCGCAGGGCGCGTGCGGCGACATCTACAAC (SEQ ID NO: 52) |
| | I403C-2 | GTTGTAGATGTCGCCGCACGCGCCCTGCGACGA (SEQ ID NO: 53) |
| Trp | I403W-1 | TCGTCGCAGGGCGCGTGGGGCGACATCTACAAC (SEQ ID NO: 54) |
| | I403W-2 | GTTGTAGATGTCGCCCCACGCGCCCTGCGACGA (SEQ ID NO: 55) |
| Arg | I403R-1 | TCGTCGCAGGGCGCGCGCGGCGACATCTACAAC (SEQ ID NO: 56) |
| | I403R-2 | GTTGTAGATGTCGCCGCGCGCGCCCTGCGACGA (SEQ ID NO: 57) |

| | | |
|---|---|---|
| *In Tables 1A and 1B, "amino acid" refers to the amino acid introduced by substitution of isoleucine at position 403 in the amino acid sequence of the wild-type crotonaldehyde-crotyl alcohol dehydrogenase via site-directed mutagenesis using the corresponding primer set. | | |

Each amino acid mutation was introduced by site-directed mutagenesis using PCR techniques.

A plasmid (pCAMO-6-Fonti) carrying a wild-type crotonaldehyde-crotyl alcohol dehydrogenase gene from *Fonticella tunisiensis* was used as a template DNA. PCR reactions were performed using each of the 19 primer combinations shown in Table 1 to amplify the full-length plasmid DNA. Each PCR reaction product was treated with restriction enzyme DpnI (37°C, 1 hour) to cut and remove the plasmid DNA used as the template. E*scherichia coli* strain JM109 was transformed separately with an aliquot of each DpnI-treated plasmid solution by heat shock method, applied to LB medium containing 50 µg/mL kanamycin, and cultured at 37°C for 24 hours.

Strains grown on the LB medium were individually inoculated into 5 mL of liquid LB medium containing 50 µg/mL kanamycin per test tube using a platinum loop, and cultured with shaking at 37°C. Plasmid DNA was extracted from each culture liquid. The analysis of the nucleotide sequences of the genes inserted in the plasmids was outsourced to Eurofins Genomics K.K. and performed by Sanger method. The results confirmed that target amino acid mutations occurred.

*Escherichia coli* strain JM109 was transformed separately with each obtained plasmid by electric pulse method (electroporation), seeded onto solid LB medium containing 50 µg/mL kanamycin, and cultured at 37°C for 24 hours.

### (2) Measurement of crotonaldehyde-crotyl alcohol dehydrogenase activity of enzymes produced by Escherichia coli transformants

*Escherichia coli* transformants grown on the solid LB medium were individually inoculated onto liquid LB medium containing 50 µg/mL kanamycin using a platinum loop, and cultured with shaking at 37°C for 24 hours. The bacterial cells were collected separately from 2 mL of each transformant culture liquid by centrifugation (4°C, 5,000 g, 10 minutes). The bacterial cells were suspended in 300 µL of reaction buffer (100 mM Tris-HCl (pH 7.5), 5 µM FeSO₄, 0.2 mM DTT), disrupted by sonication, and then centrifuged (4°C, 20,000 g, 10 minutes) to collect the disrupted cell supernatants. Similarly, disrupted cell supernatant was obtained from *Escherichia coli* strain JM109 into which pCAMO-6-Fonti containing a wild-type crotonaldehyde-crotyl alcohol dehydrogenase gene from *Fonticella tunisiensis* was introduced.

These disrupted cell supernatants were used as crude enzyme solutions to measure crotonaldehyde-crotyl alcohol dehydrogenase activity. Reaction buffer was added to each crude enzyme solution, 10 mM NADH was added as a coenzyme, and 4 mM crotonyl-CoA was added as a substrate. The mixtures were reacted at 52°C for 30 minutes. Each reaction mixture was analyzed with a gas chromatograph (Shimadzu Corporation: GC-2014, Polar-WAX column). The produced crotyl alcohol was identified by detecting a peak having a column retention time identical to that of the standard.

In the graph of Fig. 2, the relative values of the crotyl alcohol peak areas obtained from the mutant enzymes are shown, with the crotyl alcohol peak area obtained from the wild-type enzyme set to 1. Crotyl alcohol-producing activity was increased three- to more than twenty-fold in 12 mutant enzymes, which were a serine mutant (I403S), a glycine mutant (I403G), a methionine mutant (I403M), an alanine mutant (I403A), a proline mutant (I403P), a glutamine mutant (I403Q), a phenylalanine mutant (I403F), a leucine mutant (I403L), a tryptophan mutant (I403W), a lysine mutant (I403K), a cysteine mutant (I403C), and a glutamic acid mutant (I403E), as compared with the wild-type enzyme (Ile:WT). It was demonstrated that I403 plays a crucial role in crotonaldehyde-crotyl alcohol dehydrogenase activity.

### (3) Transformants of Hydrogenophilus thermoluteolus

### (3-1) Gene transfer

*Hydrogenophilus thermoluteolus* strain TH-1 was transformed separately with empty vector plasmid pCAMO-6, a plasmid expressing the wild-type crotonaldehyde-crotyl alcohol dehydrogenase from *Fonticella tunisiensis*, and plasmids expressing the mutant enzymes I403S, I403G, I403M, I403A, I403P, I403Q, I403F, and I403L by electric pulse method (electroporation), applied to solid LB medium containing 50 µg/mL kanamycin, and cultured at 52°C for 24 hours. Strains grown on the solid LB medium were individually inoculated onto solid LB medium containing 50 µg/mL kanamycin using a platinum loop, and cultured at 52°C for 24 hours. PCR was performed on the grown strains on the solid LB medium to confirm amplification of the fragments inserted in the plasmids.

The results showed that DNA fragments with lengths corresponding to the respective genes were amplified.

### (3-2) Evaluation of crotyl alcohol-producing ability of enzymes produced by transformants

The transformed strains of *Hydrogenophilus thermoluteolus* prepared as described above were individually inoculated into liquid A medium containing 50 µg/mL kanamycin using a platinum loop, and cultured with shaking for 24 hours while a gas mixture of H₂:O₂:CO₂ = 7.5:1:1.5 was supplied. The bacterial cells were collected separately from 2 mL of each culture liquid by centrifugation (4°C, 5,000 g, 10 minutes). The bacterial cells were suspended in 300 µL of reaction buffer (100 mM Tris-HCl (pH 8.5), 5 µM FeSO₄, 0.2 mM DTT), disrupted by sonication, and then centrifuged (4°C, 20,000 g, 10 minutes) to collect the disrupted cell supernatants.

The disrupted cell supernatants were subjected to polyacrylamide gel electrophoresis (SDS-PAGE). The image of the gel is shown in Fig. 3(A). The band at approximately 95 kDa, which is indicated by the arrow in the figure, represents crotonaldehyde-crotyl alcohol dehydrogenase. The eight transformants carrying mutant genes produced crotonaldehyde-crotyl alcohol dehydrogenase at levels comparable to that in the transformant carrying the wild-type crotonaldehyde-crotyl alcohol dehydrogenase gene.

The disrupted cell supernatants were used as crude enzyme solutions to evaluate crotyl alcohol-producing ability. Reaction buffer was added to each crude enzyme solution, 10 mM NADH was added as a coenzyme, and 1 mM crotonyl-CoA was added as a substrate. The mixtures were reacted at 52°C. Each reaction mixture was analyzed with a gas chromatograph (Shimadzu Corporation: GC-2014, Polar-WAX column). The produced crotyl alcohol was identified by detecting a peak having a column retention time identical to that of the standard.

Chromatograms of products from the reactions of crotonyl-CoA with the crude enzyme solutions from each transformant are shown in Fig. 3(B). NC indicates the negative control strain transformed with empty vector pCAMO-6, and Std indicates 1 mM crotyl alcohol. Crotyl alcohol was not produced (below the limit of detection) from crotonyl-CoA as a substrate using the crude enzyme solution produced by the transformant of *Hydrogenophilus thermoluteolus* strain TH-1 carrying a wild-type crotonaldehyde-crotyl alcohol dehydrogenase gene. In contrast, crotyl alcohol was produced from crotonyl-CoA as a substrate using the crude enzyme solutions produced by the eight transformants carrying mutant enzyme genes (I403S, I403G, I403M, I403A, I403P, I403Q, I403F, and I403L).

### (3-3) Production of crotyl alcohol by transformants

The transformant producing the wild-type enzyme, the eight transformed strains producing the respective mutant enzyme genes, and the NC strain with an empty vector, all prepared as described above, are individually inoculated into medium containing 50 µg/mL kanamycin, and cultured with shaking while a gas mixture of H₂, O₂, and CO₂ is supplied. After that, culture supernatants are collected by centrifugation (4°C, 5,000 g, 10 minutes). In the culture supernatants from the transformed strains expressing the respective mutant enzymes, crotyl alcohol is produced, but not in the culture supernatant from the strain with empty vector or the transformed strain producing a wild-type crotonaldehyde-crotyl alcohol dehydrogenase.

The *Hydrogenophilus* bacterium transformant of the present invention can be prepared by referring to the description of "Examples" of this specification. Other strains mentioned in this specification are publicly available, as they are internationally deposited under the Budapest Treaty, or are possessed by organizations that furnish the strains without any restriction, or are marketed, or can be prepared by those skilled in the art based on this specification.

SEQ ID Nos: 1 to 15 are shown below.
[Chem. 1]
   SEQ ID NO: 1 (gene encoding a wild-type crotonaldehyde-crotyl alcohol dehydrogenase from *Fonticella tunisiensis*) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGATAGAATT
GAAAAATTTGGTGCGACTATGAAGACAGGAAGAACAATAATCAATATGCCTTCGTCCCAA
GGTGCAATAGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 2]
   SEQ ID NO: 2 (wild-type crotonaldehyde-crotyl alcohol dehydrogenase from *Fonticella tunisiensis*) 
MKVTNAEELRLKLEEVRKAQKIFASYTQEQVDEIFRQAAMAANNARIKLAKMAVEETGMG

MKVTNAEELRLKLEEVRKAQKIFASYTQEQVDEIFRQAAMAANNARIKLAKMAVEETGMG
IVEDKVIKNHFASEYIYNKYKDEKTCGVIDRDESFGIMRVAEPIGVIAAIVPTTNPTSTA
IFKALIALKTRNGVIFSPHPRAKKSTIAAAKIVLDAAVKAGAPEGIIGWIDEPTVELSQI
VMKEADMILATGGPGMVRAAYSSGKPAIGVGAGNTPAIIDETAHIKMAVSSILLSKTFDN
GVICASEQSVIVMEQIYDEVKREFEERGAYFLKGEEIDKVRKIILVNGSINANIVGQPAH
KIAEMAGVKVPETAKVLIGEVESVELDEPFSHEKLSPVLAMYKAKTFDEALQKAARLVEL
GGFGHTSVLYTDQVKSRDRIEKFGATMKTGRTIINMPSSQGAIGDIYNFRLAPSLTLGCG
TWGGNSVSENVGVKHLLNIKSVAERRENMLWFRVPEKIYFKYGCLPVALKELKDMGKKRA
FIVTDKVLYNLGFIDTVTKTLDEIGIEYSVFFDVEPDPTLATARKGAAQMKSLNPDVIIA
VGGGSPMDAAKIMWVLYEHPEVRFEDLAMRFMDIRKRVYQFPKMGEKAMMVSIPTSSGTG
SEVTPFAVITDEKTGIKYPLADYELTPDMAIVDAELMLNMPKGLTAASGIDALTHAIEAY
VSVLASEYTNGLALEAIRLIFKYLPQAYNDGPNNIKAREKMAHAATMAGMAFANAFLGVC
HSMAHKLGAMHHVPHGVANGLLINEVIRFNAVDNPRKQTAFPQYKYPNAKFRYARIADYL
NLGGNTEDEKIELLIKAIDDLKNQVNMPKTISEAGVSREKFYATLDEMCEQAFDDQCTGA
NPRYPLISEIKQMYINAFEGISKETEENNK
[Chem. 3]
   SEQ ID NO: 3 (mutant gene I403S) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCATCGGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 4]
   SEQ ID NO: 4 (mutant gene I403G) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCAGGCGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 5]
   SEQ ID NO: 5 (mutant gene I403M)(the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCAATGGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 6]
   SEQ ID NO: 6 (mutant gene I403A) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCAGCGGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 7]
   SEQ ID NO: 7 (mutant gene I403P) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGATAGAATT
GAAAAATTTGGTGCGACTATGAAGACAGGAAGAACAATAATCAATATGCCTTCGTCCCAA
GGTGCACCGGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAATAATAAATAA
[Chem. 8]
   SEQ ID NO: 8 (mutant gene I403Q) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCACAGGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 9]
   SEQ ID NO: 9 (mutant gene I403F) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCATTCGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 10]
   SEQ ID NO: 10 (mutant gene I403L) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGATAGAATT
GAAAAATTTGGTGCGACTATGAAGACAGGAAGAACAATAATCAATATGCCTTCGTCCCAA
GGTGCACTCGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 11]
   SEQ ID NO: 11 (mutant gene I403W) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCATGGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 12]
   SEQ ID NO: 12 (mutant gene I403K) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCAAAAGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 13]
   SEQ ID NO: 13 (mutant gene I403C) (the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGGATAGAATT
GAAAAATTTGGTGCGACTATGAAGACAGGAAGAACAATAATCAATATGCCTTCGTCCCAA
GGTGCATGCGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 14]
   SEQ ID NO: 14 (mutant gene I403E)(the underlined nucleotides differ from the corresponding nucleotides of SEQ ID NO: 1) 
ATGAAAGTAACAAATGCAGAAGAACTAAGATTAAAGCTTGAGGAGGTTAGAAAAGCCCAA
AAAATATTCGCCTCCTACACCCAGGAACAGGTAGATGAAATATTTAGACAGGCTGCTATG
GCTGCTAACAATGCAAGAATTAAGCTTGCAAAGATGGCTGTTGAAGAAACAGGAATGGGA
ATTGTAGAAGACAAAGTTATTAAAAACCACTTTGCCTCAGAATATATATACAATAAATAT
AAGGATGAAAAGACCTGCGGAGTTATTGACAGAGATGAATCCTTCGGCATAATGAGGGTT
GCAGAACCTATAGGAGTTATCGCTGCAATAGTACCAACTACCAACCCGACATCCACTGCA
ATATTTAAAGCATTAATCGCTTTAAAAACAAGAAACGGTGTGATTTTCTCACCCCATCCA
AGGGCTAAAAAATCAACTATAGCAGCAGCTAAAATCGTACTTGATGCTGCAGTTAAGGCC
GGTGCCCCTGAGGGAATAATAGGATGGATTGATGAACCAACGGTTGAACTTTCACAGATC
GTTATGAAGGAAGCTGATATGATACTTGCCACAGGCGGCCCCGGAATGGTAAGAGCTGCT
TATTCCTCAGGAAAACCCGCAATAGGAGTAGGTGCTGGAAATACGCCTGCAATAATAGAT
GAAACAGCACATATTAAAATGGCAGTAAGCTCCATACTTCTTTCAAAGACATTTGACAAC
GGAGTTATATGTGCTTCAGAACAGTCAGTAATTGTAATGGAACAAATATATGACGAAGTT
AAGCGTGAGTTTGAGGAAAGAGGAGCATACTTCCTAAAGGAGAGGAAATCGACAAGGTT
AGAAAGATAATACTTGTTAATGGTTCAATAAATGCAAATATAGTTGGACAGCCTGCCCAC
AAAATAGCTGAAATGGCTGGAGTTAAAGTTCCTGAAACAGCAAAGGTATTAATCGGAGAA
GTTGAATCCGTTGAACTCGATGAACCATTCTCACACGAAAAACTTTCACCTGTGCTCGCT
ATGTATAAGGCTAAAACCTTCGATGAGGCACTTCAGAAAGCTGCAAGGCTTGTGGAACTT
GGCGGATTTGGGCATACATCAGTTCTATATACCGACCAGGTAAAATCAAGGATAGAATT
GAAAAATTTGGTGCGACTATGAAGACAGGAAGAACAATAATCAATATGCCTTCGTCCCAA
GGTGCAGAAGGAGATATCTATAACTTCAGGCTTGCTCCTTCGCTAACACTCGGATGTGGT
ACATGGGGTGGAAACTCTGTATCTGAAAACGTTGGAGTTAAGCATCTTTTAAACATCAAG
AGTGTTGCTGAAAGGAGAGAAAATATGCTTTGGTTCAGAGTTCCTGAAAAGATATACTTC
AAATACGGCTGCCTGCCTGTAGCATTAAAGGAATTAAAGGATATGGGCAAGAAGAGGGCA
TTCATCGTTACTGACAAAGTTCTGTATAATTTAGGATTTATCGATACAGTTACAAAGACC
CTTGATGAAATAGGAATTGAATATAGCGTGTTCTTTGACGTTGAACCAGACCCAACACTT
GCAACTGCAAGAAAGGGTGCAGCACAAATGAAAAGCCTCAATCCGGATGTAATAATTGCA
GTTGGTGGAGGCTCACCTATGGATGCTGCAAAGATTATGTGGGTACTTTATGAGCATCCG
GAAGTAAGGTTTGAAGACCTTGCAATGAGATTTATGGATATTAGAAAGAGAGTTTACCAG
TTCCCAAAGATGGGCGAAAAGGCTATGATGGTTTCAATCCCAACTTCTTCCGGAACAGGT
TCAGAGGTTACTCCATTTGCTGTTATAACCGATGAAAAGACGGAATTAAATATCCTCTT
GCAGACTATGAGTTAACTCCTGATATGGCAATAGTAGATGCAGAACTTATGCTCAATATG
CCAAAGGGACTTACAGCAGCATCTGGTATAGACGCCCTAACTCATGCAATAGAAGCCTAT
GTTTCGGTACTTGCATCAGAGTATACTAACGGACTTGCCCTTGAAGCGATAAGACTTATA
TTCAAATATCTACCACAGGCATATAATGATGGTCCAAACAACATCAAAGCAGGGAAAAG
ATGGCCCACGCTGCAACTATGGCTGGTATGGCCTTTGCAAATGCATTCCTCGGCGTATGC
CACTCAATGGCCCACAAGCTTGGAGCAATGCATCATGTACCACACGGAGTTGCCAACGGC
CTTCTAATTAACGAAGTAATAAGATTTAATGCAGTTGATAACCCAAGAAAGCAAACTGCA
TTCCCACAGTATAAATATCCAAACGCCAAGTTCAGATATGCAAGGATTGCTGACTACCTG
AACCTTGGCGGAAATACCGAAGATGAAAAGATTGAACTCCTTATAAAGGCAATCGACGAC
TTAAAGAACCAGGTTAACATGCCAAAAACGATAAGTGAAGCTGGTGTTTCAAGAGAGAAA
TTCTACGCAACCCTTGATGAAATGTGCGAACAGGCCTTCGACGACCAGTGCACAGGTGCC
AACCCAAGATATCCGTTAATAAGCGAAATCAAGCAGATGTATATAAACGCTTTCGAAGGA
ATTTCAAAGGAAACTGAAGAAAATAATAAATAA
[Chem. 15]
   SEQ ID NO: 15 (plasmid pCAMO-6) former half 
CCCATCGACTGCACGGTGCACCAATGCTTCTGGCGTCAGGCAGCCATCGGAAGCTGTGGTATGGCTGTGCAGGTCGTAAA
TCACTGCATAATTCGTGTCGCTCAAGGCGCACTCCCGTTCTGGATAATGTTTTTTGCGCCGACATCATAACGGTTCTGGC
AAATATTCTGAAATGAGCTGTTGACAATTAATCATCGGCTCGTATAATGTGTGGAATTGTGAGCGGATAACAATTTCACA
CAGGAAACAGGCCAGTTAGGCCTGCTAGCAGATCTGGAGGAGAAACGCATATGCTGATATCGGATCCGAATTCGAGCTCC
GTCGACAAGCTTGCGGCCGCACTGCAGCACCATCACCACCATCATTGATAAGATCCGGCTGCTAACAAAGCCCGAAAGGA
AGCTGAGTTGGCTGCTGCCACCGCTGAGCAATAACTCGAGGCCAATGAGGCCTTCAAATAAAACGAAAGGCTCAGTCGAA
AGACTGGGCCTTTCGTTTTATCTGTTGTTTGTCGGTGAACGCTCTCCTGAGTAGGACAAATCCGCCGGGAGCGGATTTGA
ACGTTGCGAAGCAACGGCCCGGAGGGTGGCGGGCAGGACGCCCGCCATAAACTGCCAGGCATCAAATTAAGCAGAAGGCC
ATCCTGACGGATGGCCTTTTTGCGTTTCTACAAACTCCGCGGATCTAGGTGAAGATCCTTTTTGATAATCTCATGACCAA
AATCCCTTAACGTGAGTTTTCGTTCCACTGAGCGTCAGACCCCGTAGAAAAGATCAAAGGATCTTCTTGAGATCCTTTTT
TTCTGCGCGTAATCTGCTGCTTGCAAACAAAAAAACCACCGCTACCAGCGGTGGTTTGTTTGCCGGATCAAGAGCTACCA
ACTCTTTTTCCGAAGGTAACTGGCTTCAGCAGAGCGCAGATACCAAATACTGTCCTTCTAGTGTAGCCGTAGTTAGGCCA
CCACTTCAAGAACTCTGTAGCACCGCCTACATACCTCGCTCTGCTAATCCTGTTACCAGTGGCTGCTGCCAGTGGCGATA
AGTCGTGTCTTACCGGGTTGGACTCAAGACGATAGTTACCGGATAAGGCGCAGCGGTCGGGCTGAACGGGGGGTTCGTGC
ACACAGCCCAGCTTGGAGCGAACGACCTACACCGAACTGAGATACCTACAGCGTGAGCTATGAGAAAGCGCCACGCTTCC
CGAAGGGAGAAAGGCGGACAGGTATCCGGTAAGCGGCAGGGTCGGAACAGGAGAGCGCACGAGGGAGCTTCCAGGGGGAA
ACGCCTGGTATCTTTATAGTCCTGTCGGGTTTCGCCACCTCTGACTTGAGCGTCGATTTTTGTGATGCTCGTCAGGGGGG
CGGAGCCTATGGAAAAACGCCAGCAACGCGGCCTTTTTACGGTTCCTGGCCTTTTGCTGGCCGCGGACGTAGAAAGCCAG
TCCGCAGAAACGGTGCTGACCCCGGATGAATGTCAGCTACTGGGCTATCTGGACAAGGGAAAACGCAAGCGCAAAGAGAA
AGCAGGTAGCTTGCAGTGGGCTTACATGGCGATAGCTAGACTGGGCGGTTTTATGGACAGCAAGCGAACCGGAATTGCCA
GCTGGGGCGCCCTCTGGTAAGGTTGGGAAGCCCTGCAAAGTAAACTGGATGGCTTTCTTGCCGCCAAGGATCTGATGGCG
CAGGGGATCAAGATCCGATCAAGAGAGGCCACTAAGGCCCAGGATGAGGATCGTTTCGCATGATTGAACAAGATGGATTG
CACGCAGGTTCTCCGGCCGCTTGGGTGGAGAGGCTATTCGGCTATGACTGGGCACAACAGACAATCGGCTGCTCTGATGC
CGCCGTGTTCCGGCTGTCAGCGCAGGGGCGCCCGGTTCTTTTTGTCAAGACCGACCTGTCCGGTGCCCTGAATGAACTAC
AGGACGAGGCAGCGCGGCTATCGTGGCTGGCCACGACGGGCGTTCCTTGCGCAGCTGTGCTCGACGTTGTCACTGAAGCG
GGAAGGGACTGGCTGCTATTGGGCGAAGTGCCGGGGCAGGATCTCCTGTCATCTCACCTTGCTCCTGCCGAGAAAGTATC
CATCATGGCTGATGCAATGCGGCGGCTGCATACGCTTGATCCGGCTACCTGCCCATTCGACCACCAAGCGAAACATCGCA
TCGAGCGAGCACGTACTCGGATGGAAGCCGGTCTTGTCGATCAGGATGATCTGGACGAAGAGCATCAGGGGCTCGCGCCA
GCCGAACTGTTCGCCAGGCTCAAGGCGCGTATGCCCGACGGCGAGGATCTCGTCGTGACTCATGGCGATGCCTGCTTGCC
GAATATCATGGTGGAAAATGGCCGCTTTTCTGGATTCATCGACTGTGGCCGGCTGGGTGTGGCGGACCGCTATCAGGACA
TAGCCTTGGCTACCCGTGATATTGCTGAAGAGCTTGGCGGCCAATGGGCTGACCGCTTCCTCGTGCTTTACGGTATCGCC
GCTCCCGATTCGCAGCGCATCGCCTTCTATCGCCTTCTTGACGAGTTCTTCTGAGGCCATCTAGGCCATGAGTAAGTCTG
ACGGAACGGAAAGTTGTCGGTTACCAGCAACGTTTCGCCCTGTTGGCCTCAAAGTTCGAGTCCGTTATTGGTATTTCCA
TACCAACTCAAACGGGATATAATGTGACATGTAACATTTCTCGATTGAGGAACGACAATGACAGTACCGGTCAAAGCGCG
TGTTCAAAAGTATCGCGACGCGCTGCGCCGCTCGGGTTTGCGTCCGGTTCAGATATGGGTGCCGGATACCCGCCGCCCGG
GCTTTGCACAAGAGTGTCGCCGCCAGTGCCTGCTCGTGACCCAGGCAGACGCCGCTGATACGCAACTCCAACGACTCATG
GATGAAGCCTTGGCAGAGATTGATGGCTGGCAGGCATGATGCGCGGCGATTTGGTAACGATCGTTACCCAGGGCGACTTT
GGCAAACCAAGGCCAGCGTTGGTGATCCAGTCCGATCTGTTCAACGAGCTCAGCAGCATAACCGTCTTTCCTGTAACCAG
[Chem. 16]
   SEQ ID NO: 15 (plasmid pCAMO-6) latter half

TACCATTGTGGATGCCCCGTTGTTGCGCGTCACGGTTGAGCCAACCCCGGAAAACGGCTTGCACAAGCGATCACAGGTGA
TGGTGGACAAAATTATGACCGTGCGGCGGGACAAAGTCGGTCAGATCATTGGACGTATCGATACGACTACGCTGACAACG
ATCGAGCGCTGCTTAGCTGTCTTCTTGGGAATCGCGAAATAAGGCCATGAAGGCCTTACCCATCACCGTGCATCACGCCG
ACCGCTTCGCCACTTTGCGACAAGAAAGCAATCTGAACGCGCTGGCGAAAATGGATCGGCGCTGCCCGGTCTACCTGTGA
AGTTCGCGCAAACGTCCATCGAGACGTACATTGCCGTTGGCATCGATCTCGACGGCGATTTCCTCTGGCTACCGATCGCG
CGCACCGCTTACGCGCCCTCGACGTAGTCGAGAAAAGCGTGATTGCGTATGTTGCGCCAACTGAGCCCGTCGGTGCGTTG
TGACCACTCGTTGTAGACCACGCCCAAAACCATCACCCCAAACGGTGCGGAAAGTGCTACTCTTTCGTCTTGCCACAACA
AGAAGGAGGGCGGGTGGTGATCGAAACCGGTGCTTTGAGAGCCAAAGAAGACCCCATTGCCTGGGTCCAACAGAAAGCAG
CCGAGGCGTTGCAACGCCTAGATCAAAAGATGGCCTCTCAGCCCGTGCAGATGTACCTACCGGGGTTCGATCTAGGGGCC
TTTCCCAACCATGTGAACCGCTCCTCGCTCTTTGCGCCTATTGCGCGGTCGCCGCAAATTTCATCGACAAACGAAGAT
GGTCAGCCGCTCGGATGCTGTGATCGAATACACTGGAGAGCAGCTCGACGAAGCCGACTGCGACCTCATGATGGCGCTCA
TCTGGTTTGCCCAGAAAAAACCGTTAGGGGAGTGGGTGACATTGAACCGAGCGCAGCTGTTGCGGCGGATCGGGCGCGCG
ACGGGCAAGCAGAACTACGAGTGGCTCCATCGCAGAATGCGGGCGATGACCGAAGCGACACTCTACATCGAAGTGCGCAA
ATCCGACGGCAGCACACGCTACAAGATCGGCTACGCCGAGGCGTTTCACCTCATCAGCGGCTTTGCGTTCGATGAGAAAA
GCGACGAATACTGCTACCAACTCGATCCCCGCTGGGTACAGCTCTTCAGCAACCGCGAATACAGCCTGATCAACTTCGAC
TTGCGATTGCAGATCAAACGGGGTCAGGACATGGCGAAAACCCTGCAACGCCTCGTTGCTACTTCTTCCGACCCCATCCA
GTCTTACGAACTCGAATGGCTCAAGAGCAAAATGGAATACACCGGAAGAATGCGCGATTTCAAAGAGTCGCTCTCCAAGG
CGTGCAATGAGCTGAAACGGTTGCACATCGTCAGTGAATACAAGATCACTCTGGACAAAAAGAAGCAACGGGAGCTGTTG
AAGCTCTGGATTCCCAAAGCGATCACGGCATAAAAGCGGTGCGACACTGCACGATCAGAAAGTATTTTCAGTTACTCAAA
CACCGTTTTTTGTTGAAAAACAACGAATTGTCATCGGTATAGCGTCGCGCACCCGAGACGACCGACGGTGTCTGGCCATC
GGTATAGCGTCGCGCTTTCTCCACCTCTTGAACAGTCGTTCGGCATCGGTATAGCGTCGCGCCTGCATCGGTATAGCGTC
GCGCTTTTTCGGTATAGCGTCGCGCTTTTTCGGTATAGCGTCGCGCGGTTGTCAAAAAATCTATTATTATTCAATAAGTT
ACAAGCGGTTTTTGACCGTCTACTATATTTCTACTTATTTCTACTAGGAAGCGCCTGTGGATAACTTCGCTCTTTTCTAC
ACAGCCCCTCAGCGGCGCAAGCGCGCCGCTGGGTAGAAAATTCCTATCGCAACGAACTGTCGTTCCTAAAAATAGGCTAC
TCGGTAAAACAACCGTATAAATGATGCCGCGTGCTCGGAGTCAACTCCAGAACGGCATCGGTATAGTGTCGCACTTAGCA
ATGTGCGAATATTTCATCGTTGAGCCGACTTTGGAGATTGGTCATGGAGATCCTCGGGTTGTCGAAGCCTTGGACTCC
TGTTGATAGATCCAGTAATGACCTCAGAACTCCATCTGGATTTGTTCAGAACGCTCGGTTGCCGCCGGGCGTTTTTTATT
GGTGAGAATCCAGTCTAGA

### Industrial Applicability

The mutant crotonaldehyde-crotyl alcohol dehydrogenase of the present invention enables highly efficient production of crotyl alcohol, which serves as a raw material for producing butadiene, a high-in-demand material in rubber, resin, and other chemical product industries; therefore, the mutant crotonaldehyde-crotyl alcohol dehydrogenase contributes to improving the sustainability of chemical production.

## Claims

1. A gene encoding a crotonaldehyde-crotyl alcohol dehydrogenase mutant described in the following (1) or (2).
(1) A DNA comprising a nucleotide sequence having, in the nucleotide sequence of SEQ ID NO: 1, a nucleotide substitution resulting in a substitution of isoleucine at position 403 of SEQ ID NO: 2 encoded by SEQ ID NO: 1 with serine, glycine, methionine, alanine, proline, glutamine, phenylalanine, leucine, tryptophan, lysine, cysteine, or glutamic acid
(2) A DNA encoding a polypeptide described in the following (2A) or (2B):
(2A) a polypeptide comprising an amino acid sequence that has 90% or more identity to an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid), or
(2B) a polypeptide comprising an amino acid sequence that has 1 to 80 amino acids deleted, substituted, inserted, or added in an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid)

2. The gene according to claim 1, wherein (1a) the above (1) is a DNA comprising the following nucleotide sequence:
a nucleotide sequence having a substitution of ATA at positions 1207 to 1209 of SEQ ID NO: 1 with TCT, TCC, TCA, TCG, AGT, or AGC,
a nucleotide sequence having a substitution of ATA at positions 1207 to 1209 of SEQ ID NO: 1 with GGT, GGC, GGA, or GGG,
a nucleotide sequence having a substitution of ATA at positions 1207 to 1209 of SEQ ID NO: 1 with ATG,
a nucleotide sequence having a substitution of ATA at positions 1207 to 1209 of SEQ ID NO: 1 with GCT, GCC, GCA, or GCG,
a nucleotide sequence having a substitution of ATA at positions 1207 to 1209 of SEQ ID NO: 1 with CCT, CCC, CCA, or CCG,
a nucleotide sequence having a substitution of ATA at positions 1207 to 1209 of SEQ ID NO: 1 with CAA or CAG,
a nucleotide sequence having a substitution of ATA at positions 1207 to 1209 of SEQ ID NO: 1 with TTT or TTC,
a nucleotide sequence having a substitution of ATA at positions 1207 to 1209 of SEQ ID NO: 1 with TTA, TTG, CTT, CTC, CTA, or CTG,
a nucleotide sequence having a substitution of ATA at positions 1207 to 1209 of SEQ ID NO: 1 with TGG,
a nucleotide sequence having a substitution of ATA at positions 1207 to 1209 of SEQ ID NO: 1 with AAA or AAG,
a nucleotide sequence having a substitution of ATA at positions 1207 to 1209 of SEQ ID NO: 1 with TGT or TGC, or
a nucleotide sequence having a substitution of ATA at positions 1207 to 1209 of SEQ ID NO: 1 with GAA or GAG, and
wherein the above (2) is a DNA comprising a nucleotide sequence that has 90% or more identity to any one of the nucleotide sequences described in the above (1a) and encoding a polypeptide having crotonaldehyde-crotyl alcohol dehydrogenase activity (wherein the DNA has a deletion, substitution, insertion, or addition of a nucleotide or nucleotides at a position or positions other than positions 1207 to 1209 of the corresponding nucleotide sequence in the above (1a)).

3. The gene according to claim 1 or 2, wherein the gene is a DNA described in the following (A) or (B).
(A) A DNA comprising any one of the nucleotide sequences of SEQ ID NOS: 3 to 14
(B) A DNA comprising a nucleotide sequence that has 90% or more identity to SEQ ID NOS: 3 to 14 and encoding a polypeptide having crotonaldehyde-crotyl alcohol dehydrogenase activity (wherein the DNA has a deletion, substitution, insertion, or addition of a nucleotide or nucleotides at a position or positions other than positions 1207 to 1209 of SEQ ID NOS: 3 to 10)

4. A crotonaldehyde-crotyl alcohol dehydrogenase mutant described in the following (3) or (4).
(3) A polypeptide comprising an amino acid sequence that has a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2
(4) A polypeptide described in the following (4A) or (4B):
(4A) a polypeptide comprising an amino acid sequence that has 90% or more identity to an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid), or
(4B) a polypeptide comprising an amino acid sequence that has 1 to 80 amino acids deleted, substituted, inserted, or added in an amino acid sequence having a substitution of isoleucine at position 403 with serine, glycine, methionine, alanine, proline, glutamine, phenylalanine, leucine, tryptophan, lysine, cysteine, or glutamic acid in the amino acid sequence of SEQ ID NO: 2, the polypeptide having crotonaldehyde-crotyl alcohol dehydrogenase activity (wherein, in the amino acid sequence of the polypeptide, the amino acid at a position corresponding to position 403 of SEQ ID NO: 2 is serine, glycine, methionine, alanine, proline, glutamine, phenylalanine, leucine, tryptophan, lysine, cysteine, or glutamic acid)

5. A transformant obtained by introducing the gene according to any one of claims 1 to 3 into a bacterium.

6. The transformant according to claim 5, wherein the bacterium is a hydrogen-oxidizing bacterium.

7. A method for producing crotyl alcohol, the method comprising a step of culturing the transformant according to claim 5 or 6.
